# EUROPÄISCHE PATENTSCHRIFT

(11) **EP 1 458 389 B1**
(45) Veröffentlichungstag und Bekanntmachung des Hinweises auf die Patenterteilung: **20.02.2008**
(21) Anmeldenummer: 02764868.2
(22) Anmeldetag: 02.09.2002
(51) Int. Cl.: A61K 31/47, A61K 9/08, A61P 7/02, A61P 9/08, A61P 9/10, A61P 15/10

(54) **WÄSSRIGE LÖSUNGEN VON MOXAVERIN ZUR BEHANDLUNG DER EREKTILEN DYSFUNKTION**
AQUEOUS SOLUTIONS OF MOXAVERIN FOR THE TREATMENT OF ERECTILE DYSFUNCTION
SOLUTIONS AQUEUSES DU MOXAVERINE DESTINEES AU TRAITEMENT DU DYSFONCTIONNEMENT ERECTILE

(30) Priorität: 31.08.2001 DE 10142418
(43) Veröffentlichungstag der Anmeldung: 22.09.2004
(73) Patentinhaber: Eckert, Ralph, 66822 Lebach (DE)
(72) Erfinder: Eckert, Ralph, 66822 Lebach (DE)
(74) Vertreter: Weber, Roland
(86) Internationale Anmeldenummer: PCT/EP2002/009777
(87) Internationale Veröffentlichungsnummer: WO 2003/018016

(56) Entgegenhaltungen:
- WO-A-00/15233
- WO-A-97/21457

## Beschreibung

Die Erfindung betrifft eine wässrige Formulierung von Moxaverin sowie die Verwendung von Moxaverin zur Herstellung von Arzneimitteln.

Moxaverin ist eine Weiterentwicklung des Wirkstoffes Papaverin und wirkt als muskulotropes Spasmolytikum, welches 2,5 x wirksamer ist als die Muttersubstanz Papaverin.

Wie experimentelle Untersuchungen belegen, ist die Substanz nach oraler Applikation 10-fach weniger toxisch als Papaverin. Die muskelrelaxierende Wirkung beruht dabei auf der Hemmung der Phosphodiesterase (PDE) und dem dadurch bedingten Anstieg des zyklischen AMP Spiegels (cAMP) in den glatten Muskelzellen. Überdies bewirkt Moxaverin eine Erhöhung der Erythrozytenflexibilität und führt zur Hemmung der Thrombozytenaggregation.

Als Indikation waren bisher Spasmen und Koliken im Magen-Darm Trakt, in der Gallenblase und im Harnleiter, Menstruationskoliken, periphere und zerebrale arterielle sowie koronare Durchblutungsstörungen bekannt. Überdies kommt Moxaverin aufgrund seiner durchblutungsfördernden Eigenschaften auch bei der Behandlung vaskulärer Demenzformen zur Anwendung.

Die Behandlung mit Moxaverin ist nach klinischen Studien mit einer guten klinischen Wirksamkeit und gleichzeitiger hoher Arzneimittelsicherheit, selbst im Rahmen hochdosierter Langzeittherapien verbunden.

Moxaverin (1-Benzyl-3-ethyl-6,7-dimethoxyisochinolin, Mᵣ 307,38) kommt dabei in der Form als freie Base sowie in der Form des Hydrochlorids (Moxaverin-HCl, Mᵣ 343,9) zum Einsatz. Da die Substanz in Wasser kaum löslich ist, wird sie fast ausschließlich in ethanolischer Formulierung verabreicht. Solche ethanolischen Formulierungen weisen einen pH-Wert von ca. 3,5 bis 4 auf.

Die orale Verabreichung ethanolischer Formulierungen ist in der Regel (mit Ausnahme der Behandlung Alkoholabhängiger) unproblematisch, jedoch eignet sich die orale Therapierung, z. B. aufgrund der schlechteren Bioverfügbarkeit des applizierten Wirkstoffs gegenüber intravenöser Applikation, nicht für alle Indikationen. So ist auch die parenterale Gabe (z.B intravenös oder intramuskulär) von 0,03 bis 0,15 g Moxaverin pro Dosi in ethanolischer Lösung bekannt. Die Verabreichung solcher Injektionen ist jedoch aufgrund der Venotoxizität des unphysiologischen Lösungsmittels und des unphysiologischen pH-Wertes der Formulierung mit erheblichen Schmerzen verbunden.

Zwar lassen sich wäßrige Moxaverin-HCl Lösungen mit physiologischem pH-Wert herstellen, jedoch wiesen arzneiliche Formulierungen auf Basis solcher Lösungen aufgrund der äußerst geringen Wasserlöslichkeit von Moxaverin-HCl zu niedrige Konzentrationen von Moxaverin-HCl auf (maximal 1 mg/ml), um eine nennenswerte arzneiliche Wirkung zu entfalten. Auch ließen sich diese geringe Konzentration an Moxaverin kaum durch die Gabe größerer Volumina ausgleichen. Zudem ist die Stabilität von Wirksubstanzen in hochkonzentrierten Lösungen allgemein größer ist als in niedrigkonzentrierten. In der Form als freie Base ist Moxaverin praktisch unlöslich in Wasser (< 1 mg/ml).

Es besteht somit Bedarf an Arzneimitteln mit flüssigen Formulierungen von Moxaverin, welche gegenüber den im Stand der Technik bekannten besser verträglich sind.

Diese Aufgabe wird erfindungsgemäß durch ein Arzneimittel gelöst, welches als wirksamen Bestandteil eine wäßrige Formulierung von Moxaverin mit mindestens einem mit H₂O mischbaren Lösungsvermittler enthält.

Die Erfindung umfaßt Arzneimittel zur Behandlung des Menschen gleichermaßen wie Tierarzneimittel.

Die Erfindung beruht auf dem überraschenden Ergebnis, daß unter Einsatz mindestes eines mit H₂O mischbaren Lösungsvermittlers wäßrige, hochkonzentrierte Formulierungen von Moxaverin erhältlich sind. Das Moxaverin wird dabei mit dem mit H₂O mischbaren Lösungsvermittler versetzt, in Lösung gebracht und anschließend auf die gewünschte Konzentration eingestellt. Überraschenderweise bleibt das Moxaverin auch nach der Verdünnung mit Wasser in Lösung und behält seine glattmuskulär relaxierende Aktivität bei.

Die erfindungsgemäßen wäßrigen Formulierungen von Moxaverin sind physiologisch verträglicher als ethanolische Formulierungen vergleichbarer Konzentration, da es bei Injektion des erfindungsgemäßen Arzneimittels zu keiner ethanolbedingten schmerzhaften Gewebsschädigung kommt.

Da die Verabreichung des erfindungsgemäßen Arzneimittels keine Schmerzen verursacht, können gegenüber ethanolischen Lösungen größere Volumina an flüssigen Moxaverin Formulierungen appliziert werden, die ähnlich hohe Moxaverin Konzentrationen aufweisen wie ethanolische Lösungen. Insgesamt können so weitaus höhere Einzeldosen verabreicht werden.

Somit ist von einer deutlichen Steigerung des therapeutischen Nutzens bei den bekannten Indikationen des Moxaverins wie peripheren arteriellen oder koronaren Verschlußerkrankungen, der vaskulär bedingten Demenz, etc. auszugehen.

Zudem weist das erfindungsgemäße Arzneimittel auf Basis der neuartigen Formulierung somit ein gegenüber Arzneimitteln auf Basis der bekannten ethanolischen Formulierungen breiteres Anwendungsspektrum auf. So können hochkonzentrierte Moxaverinlösungen auf Wasserbasis oral auch gefahrlos bei Alkoholabhängigen appliziert werden. Vor allem ist neben der systemischen Gabe durch intravenöse Injektionen nunmehr auch die lokale Injektion möglich.

Das erfindungsgemäße Arzneimittel kann dabei grundsätzlich in jeder Darreichungsform vorliegen, die sich zur Verabreichung wäßriger Moxaverinlösungen eignet. So kommen beispielsweise Arzneimittel für die orale, okulare, rektale und parenterale (z. B. subkutan, intramuskulär, intravenös, intrakavernös, etc.) Applikation in Frage; die jeweilig bestgeeignete Darreichungsform hängt jedoch individuell von der Art und Schwere des zu behandelnden pathologischen Zustandes ab. So kommen beispielsweise Tropfen für die orale Gabe oder Augentropfen ebenso in Frage wie wäßrige Gele oder Weichkapseln mit zumindest teilweise flüssigem Inhalt. Besonders bevorzugte Ausführungsformen sind Injektionslösungen, da die im Stand der Technik bekannten moxaverhinhaltigen Injektionslösungen hinsichtlich physiologischer Verträglichkeit einerseits und therapeutischer Wirksamkeit andererseits besonders unbefriedigend sind.

Der Begriff Moxaverin umfaßt dabei den Wirkstoff als freie Base als auch als pharmazeutisch verträgliches Salz, Ester, Amid sowie jede Art von physiologisch verträglichem Moxaverinderivat. Pharmazeutisch verträgliche Salze sind Salze, die aus pharmazeutisch verträglichen, nichttoxischen Säuren von Moxaverin hergestellt werden, bevorzugtes Beispiel ist hier Moxaverin-Hydrochlorid. Moxaverin in der Form als freie Base weist den Vorteil auf, daß der pH-Wert bei Herstellung der wäßrigen Formulierung von sich aus nicht zu niedrig ist. Bei Verwendung des Moxaverinhydrochlorids ist jedoch andererseits die Herstellung besonders stark konzentrierter Moxaverinlösungen möglich, da das Hydrochlorid von sich aus bereits in geringem Maße wasserlöslich ist.

Lösungsvermittler sind Stoffe, die die Löslichkeit anderer Stoffe verbessern. Die Lösungsvermittlung kann dabei auf Grundlage der Komplexierung, durch Molekülvariation und Salzbildung, durch Mizellenbildung (Solubilisation) oder durch Verbesserung der Lösungsbedingungen infolge struktureller Änderungen des Lösungsmittels (Cosolvatisierung) erfolgen. Erfindungsgemäß werden als Lösungsvermittler vorzugsweise Cosolventien von Wasser (d. h. mit Wasser beliebig mischbare organische Lösungsmittel) mit Ausnahme des Ethanols eingesetzt. Als Lösungsvermittler können dabei grundsätzlich alle zur Herstellung von Arzneimitteln geeigneten Lösungsvermittler eingesetzt werden, in denen Moxaverin gut lösbar ist (d. h. besser lösbar ist als in Wasser, vorzugsweise mindestens ähnlich gut lösbar wie in Ethanol) und die mit Wasser mischbar sind.

Gemäß einer bevorzugten Ausführungsform sind in der wäßrigen Lösung mindestens 0,5 mg/ml, vorzugsweise wenigstens 1 mg/ml Moxaverin enthalten. Solche Konzentrationen sind herkömmlich nur in ethanolischer Lösung erhältlich, nicht in gewöhnlicher wäßriger Lösung.

Gemäß einer bevorzugten Ausführungsform wird als Lösungsvermittler ein oder eine Mischung verschiedener Polyalkylenglykole eingesetzt. Insbesondere eignen sich hierbei aufgrund ihrer physiologischen Verträglichkeit Polyalkylenglykole aus der Gruppe der Polypropylenglykole oder Polyethylenglykole. Hierbei ist der Einsatz von Polyethylenglykolen bevorzugt. Polyethylenglykole zeichnen sich durch ihre gute Verträglichkeit und ihrer hervorragenden Lösungseigenschaften bei unbegrenzter Mischbarkeit mit Wasser aus.

Zur Herstellung des erfindungsgemäßen Arzneimittels kommen dabei insbesondere Polyethylenglykole mit mittleren Molekulargewichten (Gewichtsmittel) zwischen 200 und 2000 in Frage, da diese bei Raumtemperatur im wesentlichen flüssig sind. Dabei ist mit Polyethylenglykolen mit einem mittleren Molekulargewicht von 200 (PEG 200) bis 400 (PEG 400) die Herstellung besonders hochkonzentrierter wässriger Moxaverinlösungen möglich. Demgemäß ist die Verwendung von PEG 400 als Lösungsvermittler besonders bevorzugt.

Aufgrund ihrer guten Lösungseigenschaften bei guter Verträglichkeit kann auch der Einsatz wäßriger Polyvinylpyrrolidon (PVP) Lösungen mit PVP-Konzentrationen, die zur Herstellung arzneilicher Lösungen geeignet sind, als Lösungsvermittler zweckmäßig sein.

Aufgrund der maximalen Löslichkeit von ca. 40 mg/ml Moxaverin in unverdünntem PEG und der zulässigen Obergrenzen von 30 % PEG in Arzneimitteln ist die in der fertigen wäßrigen arzneilichen Lösung maximal erreichbare Moxaverinkonzentration bei Verwendung von PEG als Lösungsvermittler bei ca. 12 mg/ml anzusiedeln.

In dem erfindungsgemäßen Arzneimittel können neben den genannten Stoffen auch weitere übliche Formulierungs- und Zusatzstoffe (abhängig von der jeweiligen Form des Arzneimittels und der jeweiligen Indikation) enthalten sein. Da der Einsatz einer möglichst geringen Anzahl von Zusatzstoffen, die Gefahr von Nebenwirkungen minimiert, weist das erfindungsgemäße Arzneimittel eine möglichst geringe Anzahl von Zusatzstoffen auf. Diesem Zweck kommt es ebenso zugute, wenn die wäßrige Formulierung des erfindungsgemäßen Arzneimittels nur eine einzigen Lösungsvermittler, vorzugsweise Polyethylenglykol (insbesondere PEG 400) enthält.

Um die Gefahr unerwünschter Wirkungen zu minimieren und möglichst dünnflüssige Formulierungen zu erhalten, ist es zweckmäßig, wenn der Gehalt an Lösungsvermittler in der fertigen wäßrigen Formulierung möglichst niedrig ist. Der Gehalt an Polyethylenglykol liegt daher in der wäßrigen Formulierung zweckmäßigerweise bei maximal 30 Vol.%, vorzugsweise jedoch darunter.

Es kann dabei - je nach Indikation - zweckmäßig sein, daß im erfindungsgemäßen Arzneimittel neben Moxaverin ein oder mehrere weitere arzneilich wirksame Bestandteile enthalten sind. Die arzneilich wirksamen Bestandteile können dabei (z. B. im Fall von Gelkapseln, welche unter anderem eine wäßrige Formulierung von Moxaverin enthalten) in einer anderen als der wäßrigen Formulierung enthalten sein. Vorzugsweise sind sie jedoch ebenfalls in der wäßrigen Formulierung enthalten. Als arzneilich wirksame Bestandteile kommen dabei grundsätzlich alle Substanzen in Frage, welche bei Applikation von Moxaverin nicht kontraindiziert sind und arzneiliche Wirkungen entfalten, die sich additiv oder synergistisch zur Wirkung des Moxaverins verhalten. Dies können Substanzen mit anderer pharmakologischer Wirkung sein als die des Moxaverins (im Sinne einer Ergänzung), aber auch solche mit im wesentlichen gleicher oder ähnlicher pharmakologischer Wirkung (z. B. antihypertensive und/oder durchblutungsfördernde Wirkstoffe). Bevorzugterweise werden eine oder mehrere Substanzen aus der Gruppe α-Rezeptorantagonisten (a-Rezeptorblocker) der PDE-Inhibitoren und/oder der Ca-Antagonisten als weitere arzneilich wirksame Bestandteile eingesetzt.

Gemäß einer besonders bevorzugten Ausführungsform des Arzneimittels weist die wäßrige Formulierung einen im wesentlichen physiologischen pH-Wert von zwischen 6,0 bis 8,0, vorzugsweise zwischen 7,0 und 8,0, besonders bevorzugt zwischen 7,3 und 7,5 und insbesondere von ca. 7,4 auf. Solche Formulierungen mit im wesentlichen physiologischem pH-Wert zeichnen sich durch eine besonders gute Verträglichkeit bei parenteraler Gabe aus, deren Injektion nicht mit pH-bedingter Venotoxizität verbunden ist. Die Herstellung solcher wäßrigen Formulierungen mit im wesentlichen physiologischen pH erfolgt durch pH-Einstellung mit einem herkömmlichen, zur Herstellung arzneilicher Lösungen geeigneten physiologischen Puffer nach Lösung des Moxaverins und anschließende Verdünnung auf die gewünschte Endkonzentration. Es hat sich dabei überraschend herausgestellt, daß Moxaverin in dem erfindungsgemäßen Arzneimittel sowohl in seiner Form als freie Base als auch in seiner Form als Hydrochlorid auch nach Einstellung eines im wesentlichen physiologischen pH-Wertes noch stabil und aktiv ist und nicht ausfällt.

In dieser Form ist das erfindungsgemäße Arzneimittel für den Einsatz im Rahmen eines besonders großen Spektrums unterschiedlicher Indikationen geeignet. So sind nunmehr neben der oralen Gabe auch Injektionen von Moxaverin schmerzfrei möglich, zum Beispiel systemisch intravenöse, aber auch lokale wie die intrakavernöse Injektion. Überdies können solche wäßrigen, pH-neutralen Formulierungen von Moxaverin auch als Augentropfen zum Einsatz kommen, ohne zu einer lokalen Reizung zu führen.

Die jeweilige Konzentration an Moxaverin im Arzneimittel und die pro dosi verabreichte Gesamtmenge an Moxaverin hängt im einzelnen von der jeweiligen Darreichungsform (Gelkapsel, Augentropfen, Injektionslösung etc.), Indikation und der Schwere des zu behandelnden pathologischen Zustandes ab. Für die intravenöse systemische Gabe von Moxaverin, z. B. im Rahmen der Schlaganfalltherapie, sind hohe Einzeldosen erwünscht mit Konzentrationen von 12 mg/ml Moxaverin und mehr. Bei der intrakavernösen Schwellkörperinjektionstherapie sind Einzeldosen von vorzugsweise 1 bis 10 mg, appliziert in einem möglichst kleinem Volumen, z. B. in 1 ml Injektionslösung, zweckmäßig.

Gemäß einer besonders zweckmäßigen Ausführungsform weist die wäßrige Formulierung des erfindungsgemäßen Arzneimittels eine im wesentlichen isotonische und vorzugsweise isotonische Einstellung auf (d. h. eine zumindest im wesentlichen isotonische Elektrolytkonzentration und/oder eine zumindest im wesentlichen isotonische Elektrolytzusammensetzung). Dies kann beispielsweise durch Einstellung mit Kochsalz, mit herkömmlichen Tyrode-Puffer oder anderen, zur Herstellung arzneilicher Lösungen geeigneter Puffern erfolgen, wie dies dem Fachmann bekannt ist.

Zur Herstellung der wäßrigen Formulierung des erfindungsgemäßen Arzneimittels ist ein Verfahren geeignet, in dem das oder die arzneilich wirksamen Bestandteile in unverdünntem Lösungsvermittler, vorzugsweise in Polyalkylenglykol und besonders bevorzugt in Polyethylenglykol gelöst werden, woran sich die Zugabe eines geeigneten Puffers zur Einstellung eines im wesentlichen physiologischen pH-Wertes und/oder eines im wesentlichen isotonischen Elektrolytgehaltes bezogen auf die Endverdünnung der wäßrigen Formulierung, sowie die Zugabe von H₂O oder wäßriger Pufferlösung auf das Endvolumen anschließen.

Es hat sich herausgestellt, daß die Herstellung der wäßrigen Lösung in unverdünntem Polyethylenglykol im Temperaturbereich zwischen 20 und 40°C und vorzugsweise bei ca. 37°C besonders zweckmäßig ist. Bei diesen Temperaturen ist löst sich Moxaverin besonders gut in Polyethylenglykolen und hier vor allem auch Polyethylenglykole im Bereich mittleren relativen Molekulargewichts zwischen 200 und 600 zutrifft.

Das Arzneimittel eignet sich aufgrund der wäßrigen Formulierung seines wirksamen Bestandteils grundsätzlich für alle Applikationen. Im Gegensatz zu flüssigen Formulierungen auf unphysiologischer Lösungsmittelbasis eignet es sich jedoch darüber hinaus insbesondere auch für Applikationsformen, für die flüssige Formulierungen von Moxaverin auf Ethanolbasis ungeeignet oder mit Nachteilen verbunden sind.

Durch die mit der neuen Galenik verbundenen Vorteile gegenüber herkömmlichen wäßrigen oder ethanolischen Formulierungen von Moxaverin, ermöglicht das erfindungsgemäße Arzneimittel den Einsatz von Moxaverin im Rahmen neuer Indikationen. So ist es aufgrund der verbesserten Eigenschaften gegenüber bekannten moxaverinhaltigen Arzneimitteln nun möglich, Moxaverin beispielsweise zur Behandlung erektiler Funktionsstörungen lokal einzusetzen, aber auch intraokular zur Verbesserung der Netzhautdurchblutung, insbesondere bei Diabetikern, Hypertonikern und MS-Patienten.

Zur Therapierung der erektilen Dysfunktion mit Moxaverin ist es notwendig, Moxaverin lokal zu applizieren. Besonders wirksam sind hier lokale Injektionen, z. B. intrauretrale und insbesondere intrakavernöse. Da kein beliebig großes Volumen intrakavernös injiziert werden kann, muß zur Verabreichung einer ausreichenden absoluten Menge an Moxaverin eine Mindestkonzentration von 0,5 mg/ml, vorzugsweise jedoch 1 mg/ml Moxaverin in der Formulierung vorliegen Mit den herkömmlichen wäßrigen Formulierungen lassen sich solche Konzentrationen nicht erreichen. Die hochkonzentrierten ethanolischen Formulierungen eignen sich dagegen aufgrund der durch ihre Injektion verursachten Schmerzen nicht zur intrakavernösen Applikation. Das erfindungsgemäße Arzneimittel weist demgegenüber keines dieser Nachteile auf: Gemäß einer bevorzugten Ausführungsform ist das erfindungsgemäße Arzneimittel somit zur Behandlung der erektilen Dysfunktion bestimmt.

Die Erfindung bezieht sich weiterhin auf die vorstehend beschriebenen wäßrigen Formulierungen von Moxaverin sowie deren Verwendung zur Herstellung von Arzneimitteln. Weiterer Gegenstand der Erfindung sind Injektionslösungen auf Basis der wäßrigen Formulierung von Moxaverin. Mit Hinsicht auf die Behandlung erektiler Dysfunktionsstörungen sind hierbei Injektionslösungen bevorzugt, welche lokal, z. B. zur intrauretralen und insbesondere zur intrakavernösen Injektion bestimmt sind. Zur Behandlung anderer pathologischer Zustände können demgegenüber andere Injektionslösungen, beispielsweise zur intravenösen oder intramuskulären Applikation, zweckmäßig sein.

Aufgrund der neuartigen und gegenüber bekannten Formulierungen verbesserten Eigenschaften der erfindungsgemäßen Formulierung von Moxaverin ist es nun möglich, Moxaverin bei der lokalen Behandlung der erektilen Dysfunktion einzusetzen. Die Erfindung bezieht sich demgemäß auch auf die Verwendung von Moxaverin zur Herstellung von Arzneimitteln zur Behandlung der erektilen Dysfunktion.

Gemäß einer besonders zweckmäßigen Ausführungsform weist ein solches Arzneimittel zur Behandlung der erektilen Dysfunktion als wirksamen Bestandteil eine wäßrige Formulierung mit zwischen 1 bis 10 mg Moxaverinhydrochlorid pro 1 ml Endvolumen auf und enthält weiterhin maximal 12,5 %, vorzugsweise jedoch weniger Polyethylenglykol mit mittlerem Molekulargewicht von 400 Gewichtsmittel und hat einen pH-Wert von ca. 7,4.

Im folgenden soll die Erfindung anhand von Beispielen näher erläutert werden.

### Beispiel 1:

### Herstellung einer Moxaverin-haltigen Injektionslösung

400 mg Moxaverinhydrochlorid wurden in 10 ml PEG 400 (Firma Sigma) unter ständigem Rühren auf einer Heizplatte bei 37°C gelöst. Nach vollständiger Lösung wurden 4 ml 10 x konzentrierter Tyrodelösung zugegeben und dann zur pH-Einstellung unter Rühren und ständiger pH-Kontrolle 2M NaOH so lange zugetropft, bis ein pH-Wert von 7,4 erreicht war. Anschließend wurde das Volumen mit H₂O b.d. auf 40 ml aufgefüllt. Die fertige. Stammlösung mit 10 mg/ml Moxaverin-HCl, pH=7,4 und isotonischer Elektrolytkonzentration konnte nach Sterilfiltration direkt eingesetzt oder vorher mit 1 x Tyrodelösung auf eine gewünschte Moxaverin Konzentration weiterverdünnt werden.

### Beispiel 2:

### Löslichkeit von Moxaverin-Hydrochlorid in polyethylenglykolhaltigen wäßrigen Lösungen

Moxaverin-Hydrochlorid wurde als Reinsubstanz in steigenden Masseanteilen (siehe Tabelle 1) in 10 ml handelssüblichem Polyethylenglykol mit mittlerem Molekulargewicht von 200 bzw. 400 (Firma Sigma, PEG 200 bzw. 400, < 1 % H₂O) unter ständigem Rühren bei einer Temperatur von 37°C vollständig in Lösung gebracht. Hierbei ließen sich in unverdünntem Polyethylenglykol 400 Moxaverinkonzentrationen bis zu 40mg/ml erzielen. (Zum Vergleich: In EtOH lassen sich maximal 200 mg/ml und in H₂O < 1 mg/ml Moxaverinhydrochlorid in Lösung bringen.)

Anschließend wurde das gelöste Moxaverin unter ständigem Rühren durch Zugabe von 1 x Tyrodelösung mit normaler extrazellulärer Elektrolytzusammensetzung in wäßrige Lösung überführt. Die Lösungen ließen sich beliebig verdünnen, ohne daß das gelöste Moxaverin ausfiel.

Zur Feststellung der pH-Abhängigkeit des Lösungsverhaltens von Moxaverinhydrochlorid wurde in einer weiteren Versuchsreihe nach der vollständigen Lösung in unverdünntem PEG durch Zugabe von NaOH ein pH-Wert von 7,4 eingestellt.

Wie der Tabelle 1 zu entnehmen ist, fiel das in PEG 200 gelöste Moxaverin-HCl bei Konzentrationen von 35mg/ml und mehr nach Neutralisierung aus. Das in PEG 400 gelöste Moxaverin dagegen blieb selbst bei hohen Konzentrationen auch nach pH-Neutralisierung in Lösung. Die so hergestellten pH-neutralen Moxaverinlösungen konnten ebenfalls beliebig verdünnt werden. Das Moxaverin fiel auch bei geringen PEG Konzentrationen von 5 % und weniger nicht aus. Das darin gelöste Moxaverin-HCl war aktiv. Die Lösungen blieben mehr als 6 Monate stabil, ohne daß das Moxaverin ausfiel.

Vergleichsweise wurde versucht, unter gleichen Bedingungen Moxaverin-HCl in mit H₂O bzw. mit Tyrodelösung verdünntem 5 -10 %igem PEG zu lösen. (D. h. es wurden, analog zu der in Tabelle 1 dargestellten Versuchsreihe, 15 bis 40 mg Moxaverin-HCl in 10 ml 10 % PEG 400 bzw. 7,5 bis 20 mg Moxaverin-HCl in 10 ml 5 % PEG 400 gegeben und bei 37°C gerührt.) Die direkte Lösung der Reinsubstanz in verdünntem PEG war jedoch weder in verdünntem PEG 400 noch verdünntem PEG 200 möglich.

### Beispiel 3

### Patientenstudie

Zwanzig Patienten mit vollständig abgeklärter Genese der erektilen Dysfunktion wurden intrakavernöse Injektionen (Schwellkörperinjektionstherapie) wäßriger Lösungen von Moxaverin verabreicht. Dabei wurden pro Einzelinjektion 1 ml einer Injektionslösung mit 1 bis 10 mg Moxaverin-Hydrochlorid pro ml Endvolumen in Tyrodepuffer mit pH = 7,4 und einer Endkozentration von 2,5 % PEG 400 (bei 1 mg Moxaverin-HCl/ml) bis 25 % PEG 400 (bei 10 mg Moxaverin-HCl/ml) verabreicht. Bei mehr als 90 % der Fälle konnte so eine ausreichend starke penile Erektion mit Erektionsgrad E = 4-5 (bei einer Skala von 0 bis 5) provoziert werden. Mit der Injektion verbundene Schmerzen wurden nicht beobachtet. Nebenwirkungen wie Priapismus waren ebenfalls nicht zu verzeichnen. Die Anwendung von Moxaverin in der erektilen Dysfunktion bewirkte in dieser Studie die Aufhebung der pathologischen Symptomatik bei gleichzeitigem Ausbleiben von unerwünschten Nebenwirkungen.

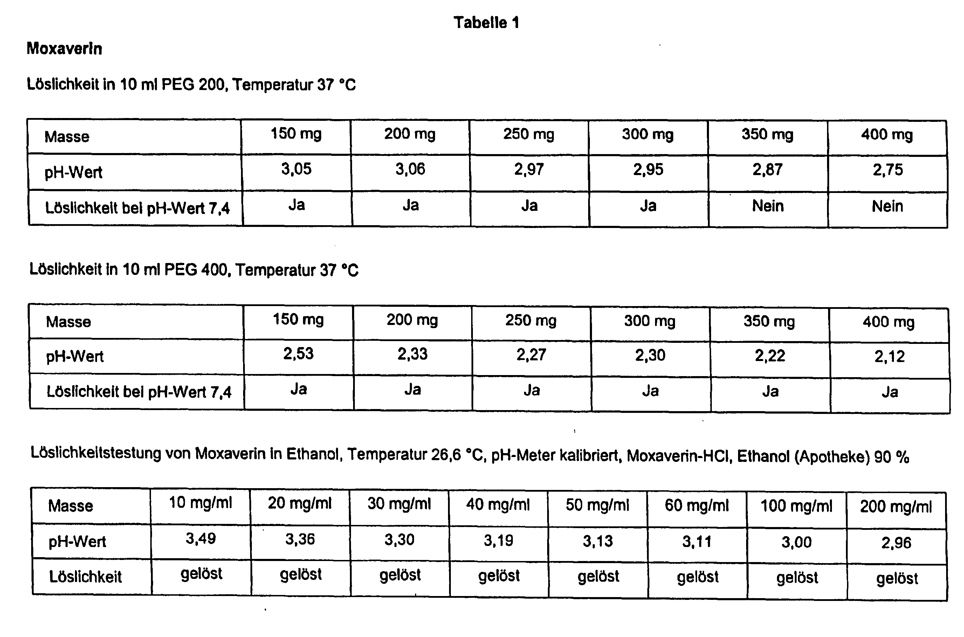

## Patentansprüche

1. Arzneimittel,
**dadurch gekennzeichnet, daß** es eine wäßrige Formulierung von Moxaverin mit mindestens einem mit H₂O mischbarem Lösungsvermittler enthält.

2. Arzneimittel gemäß Anspruch 1, **dadurch gekennzeichnet, daß** der Gehalt an Moxaverin in der wäßrigen Formulierung mindestens 0,5 mg/ml, vorzugsweise jedoch mindestens 1 mg/ml beträgt.

3. Arzneimittel gemäß einem der Ansprüche 1 oder 2, **dadurch gekennzeichnet, daß** der Gehalt an Lösungsvermittler in der wäßrigen Formulierung maximal 30 Vol.%, vorzugsweise jedoch weniger beträgt.

4. Arzneimittel gemäß einem der Ansprüche 1 bis 3, **dadurch gekennzeichnet, daß** als Lösungsvermittler ein oder mehrere Polyalkylenglykole in der wäßrigen Formulierung enthalten sind.

5. Arzneimittel gemäß Anspruch 4, **dadurch gekennzeichnet, daß** das oder die Polyalkylenglykole Polypropylenglykole und/oder Polyethylenglykole sind.

6. Arzneimittel gemäß Anspruch 5, **dadurch gekennzeichnet, daß** die wäßrige Formulierung Polyethylenglykole mit mittlerem Molekulargewicht zwischen 200 bis 2000 enthält.

7. Arzneimittel gemäß Anspruch 6, **dadurch gekennzeichnet, daß** die wäßrige Formulierung Polyethylenglykole mit mittlerem relativem Molekulargewicht von 400 enthält.

8. Arzneimittel gemäß einem der vorstehenden Ansprüche, **dadurch gekennzeichnet, daß** es einen oder mehrere weitere arzneilich wirksame Bestandteile enthält.

9. Arzneimittel gemäß Anspruch 8, **dadurch gekennzeichnet, daß** als weitere arzneilich wirksame Bestandteile ein oder mehrere Substanzen aus der Gruppe der a-Rezeptorantagonisten, PDE-Inhibitoren und/oder der Ca-Antagonisten enthalten sind.

10. Arzneimittel gemäß einem der Ansprüche 1 bis 9, **dadurch gekennzeichnet, daß** die wäßrige Formulierung einen im wesentlichen physiologischen pH-Wert aufweist.

11. Arzneimittel gemäß einem der vorstehenden Ansprüche, **dadurch gekennzeichnet, daß** die wäßrige Formulierung eine im wesentlichen isotonische Einstellung aufweist.

12. Arzneimittel gemäß Anspruch 1, **dadurch gekennzeichnet, daß** mindestens ein weiterer arzneilich wirksamer Bestandteil, gelöst in unverdünntem Lösungsvermittler bei geeigneten pH-Wert, darin enthalten ist.

13. Arzneimittel gemäß Anspruch 12, **dadurch gekennzeichnet, daß** die Herstellung bei zwischen 16 und 40 °C und insbesondere bei ca. 37 °C erfolgt.

14. Arzneimittel gemäß Anspruch 13, **dadurch gekennzeichnet, daß** ein geeigneter Puffer zur Einstellung einer isotonischen Elektrolytkonzentration zugesetzt wird.

15. Arzneimittel gemäß einem der vorstehenden Ansprüche, **dadurch gekennzeichnet, daß** es zur parenteralen Verabreicherung bestimmt ist.

16. Arzneimittel gemäß Anspruch 15, **dadurch gekennzeichnet, daß** es zur Behandlung der erektilen Dysfunktion bestimmt ist.

17. Arzneimittel gemäß Anspruch 16, **dadurch gekennzeichnet, daß** es eine wäßrige Formulierung von Moxaverin mit zwischen 0,5 und 6 mg/ml, vorzugsweise 1 bis 5 mg/ml Moxaverin und maximal 30 % PEG 400 enthält, und eine isotonische Elektrolytkonzentration sowie einen im wesentlichen physiologischen pH-Wert aufweist.

18. Arzneimittel gemäß einem der Ansprüche 1 bis 15, **dadurch gekennzeichnet, daß** es zur Behandlung peripherer arterieller oder koronarer Verschlußerkrankungen bestimmt ist.

19. Arzneimittel gemäß einem der Ansprüche 1 bis 15, **dadurch gekennzeichnet, daß** es zur Behandlung vaskulär bedingter Demenzformen bestimmt ist.

20. Wäßrige Formulierung von Moxaverin gemäß einem der vorstehenden Ansprüche 1 bis 14.

21. Injektionslösung nach einem der vorstehenden Ansprüche 15 oder 16.

22. Injektionslösung gemäß Anspruch 21 zur Verwendung bei der intrakavernösen Injektion.

23. Verwendung von Moxaverin zur Herstellung eines Arzneimittels zur Behandlung der erektilen Dysfunktion.

24. Verwendung einer wäßrigen Formulierung von Moxaverin gemäß Anspruch 20 zur Herstellung eines Arzneimittels.

25. Verwendung gemäß Anspruch 24, **dadurch gekennzeichnet, daß** das Arzneimittel ein Arzneimittel zur Behandlung peripherer arterieller oder koronarer Verschlußerkrankungen ist.

26. Verwendung gemäß Anspruch 24, **dadurch gekennzeichnet, daß** das Arzneimittel als Arzneimittel zur Behandlung vaskulär bedingter Demenzerkrankungen dient.

27. Verwendung gemäß einem der Ansprüche 23 bis 26, **dadurch gekennzeichnet, daß** das Arzneimittel eine Injektionslösung ist.

## Claims

1. Medication, **characterized in that** it contains an aqueous formulation of moxaverin with at least one solubilizer that is miscible with H₂O.

2. Medication according to claim 1, **characterized in that** the content of moxaverin in the aqueous formulation is at least 0.5 mg/ml, preferably, however, at least 1 mg/ml.

3. Medication according to one of claims 1 or 2, **characterized in that** the content of solubilizer in the aqueous formulation is a maximum of 30 vol.-%, but preferably less.

4. Medication according to one of claims 1 to 3, **characterized in that** one or more polyalkylene glycols is/are contained in the aqueous formulation.

5. Medication according to claim 4, **characterized in that** the polyalkylene glycol (s) is/are polypropylene glycols and/or polyethylene glycols.

6. Medication according to claim 5, **characterized in that** the aqueous formulation contains polyethylene glycols having an average molecular weight between 200 and 2000.

7. Medication according to claim 6, **characterized in that** the aqueous formulation contains polyethylene glycols having an average relative molecular weight of 400.

8. Medication according to one of the preceding claims, **characterized in that** it contains one or more additional medicinally active ingredients.

9. Medication according to claim 8, **characterized in that** one or more substances from the group of α -receptor antagonists (α-receptor blockers), PDE inhibitors and/or Ca antagonists is/are contained as additional medicinal ingredients.

10. Medication according to one of claims 1 to 9, **characterized in that** the aqueous formulation has an essentially physiological pH.

11. Medication according to one of the preceding claims, **characterized in that** the aqueous formulation has an essentially isotonic setting.

12. Medication according to claim 1 **characterized in that** it contains at least one further medicinally active ingredient dissolved in undiluted solubilizer at a suitable pH.

13. Medication according to claim 12, **characterized in that** the preparation is done at between 16 and 40°C, and particularly at approximately 37°C.

14. Medication according to claim 13, **characterized in that** a suitable buffer for setting any isotonic electrolyte concentration is added.

15. Medication according to one of the preceding claims, **characterized in that** it is intended for parenteral administration.

16. Medication according to claim 15, **characterized in that** it is intended for the treatment of erectile dysfunction.

17. Medication according to claim 16, **characterized in that** it contains an aqueous formulation of moxaverin with between 0.5 and 6 mg/ml, preferably 1 to 5 mg/ml moxaverin, and maximally 30% PEG 400, and that it demonstrates an isotonic electrolyte concentration as well as an essentially physiological pH.

18. Medication according to one of claims 1 to 15, **characterized in that** it is intended for the treatment of peripheral arterial or coronary occlusion disease.

19. Medication according to one of claims 1 to 15, **characterized in that** it is intended for the treatment of vascular-related dementia.

20. Aqueous formulations of moxaverin according to one of the above claims 1 to 14.

21. Injection solution according to one of the preceding claims 15 or 16.

22. Injection solution according to claim 21, for use in intravenous injections.

23. Use of moxaverin for the production of a medication for the treatment of erectile dysfunction.

24. Use of an aqueous formulation of moxaverin according to claim 20 for the production of a medication.

25. Use according to claim 24, **characterized in that** the medication is a medication for the treatment of peripheral arterial or coronary occlusion disease.

26. Use according to claim 24, **characterized in that** the medication serve as a medication for the treatment of vascular-related forms of dementia diseases.

27. Use according to one of claims 23 to 26, **characterized in that** the medication is an injection solution.

## Revendications

1. Médicament, **caractérisé en ce qu'**il contient une formule aqueuse de moxavérine avec au moins un solubiliseur miscible dans H₂O.

2. Médicament selon la revendication 1, **caractérisé en ce que** la teneur en moxavérine dans la formule aqueuse est au moins de 0,5 mg/ml, de préférence toutefois au moins de 1 mg/ml.

3. Médicament selon l'une des revendications 1 ou 2, **caractérisé en ce que** la teneur en solubiliseur dans la formule aqueuse est au maximum de 30 % en volume, de préférence toutefois moins.

4. Médicament selon l'une des revendications 1 à 3, **caractérisé en ce qu'**un ou plusieurs polyalkylènes glycols sont contenus en tant que solubiliseur dans la formule aqueuse.

5. Médicament selon la revendication 4, **caractérisé en ce que** le ou les polyalkylènes glycols sont des polyalkylènes glycols et/ou des polyéthylènes glycols.

6. Médicament selon la revendication 5, **caractérisé en ce que** la formule aqueuse contient des polyéthylènes glycols d'un poids moléculaire moyen entre 200 et 2 000.

7. Médicament selon la revendication 6, **caractérisé en ce que** la formule aqueuse contient des polyéthylènes glycols d'un poids moléculaire moyen relatif de 400.

8. Médicament selon l'une des revendications précédentes, **caractérisé en ce qu'**il contient un ou plusieurs autres ingrédients efficaces sur le plan médicamenteux.

9. Médicament selon la revendication 8, **caractérisé en ce qu'**une ou plusieurs substances du groupe des récepteurs antagonistes α, inhibiteurs PDE et/ou antagonistes Ca sont contenues en tant qu'autres ingrédients efficaces sur le plan médicamenteux.

10. Médicament selon l'une des revendications 1 à 9, **caractérisé en ce que** la formule aqueuse présente une valeur pH pour l'essentiel physiologique.

11. Médicament selon l'une des revendications précédentes, **caractérisé en ce que** la formule aqueuse présente une préparation pour l'essentiel isotonique.

12. Médicament selon la revendication 1, **caractérisé en ce qu'**au moins un autre ingrédient efficace sur le plan médicamenteux, dissout dans un solubiliseur non dilué à valeur pH appropriée, y est contenu.

13. Médicament selon la revendication 12, **caractérisé en ce que** la fabrication s'effectue entre 16 et 40 °C et en particulier à environ 37 °C.

14. Médicament selon la revendication 13, **caractérisé en ce qu'**un tampon approprié pour la préparation d'une concentration électrolytique isotonique est ajouté.

15. Médicament selon l'une des revendications précédentes, **caractérisé en ce qu'**il est destiné à l'appauvrissement parentéral.

16. Médicament selon la revendication 15, **caractérisé en ce qu'**il est destiné au traitement du dysfonctionnement érectile.

17. Médicament selon la revendication 16, **caractérisé en ce qu'**il contient une formule aqueuse de moxavérine avec entre 0,5 et 6 mg/ml, de préférence 1 à 5 mg/ml de moxavérine et au maximum 30% de PEG 400, et présente une concentration électrolytique isotonique ainsi qu'une valeur pH pour l'essentiel physiologique.

18. Médicament selon l'une des revendications 1 à 15, **caractérisé en ce qu'**il est destiné au traitement de maladies obstructives artérielles ou coronariennes périphériques.

19. Médicament selon l'une des revendications 1 à 15, **caractérisé en ce qu'**il est destiné au traitement de formes de démence d'origine vasculaire.

20. Formule aqueuse de moxavérine selon l'une des revendications 1 à 14 précédentes.

21. Solution injectable selon l'une des revendications 15 ou 16 précédentes.

22. Solution injectable selon la revendication 21 pour utilisation en injection intracaverneuse.

23. Utilisation de moxavérine dans la fabrication d'un médicament pour le traitement du dysfonctionnement érectile.

24. Utilisation d'une formule aqueuse de moxavérine selon la revendication 20 dans la fabrication d'un médicament.

25. Utilisation selon la revendication 24, **caractérisée en ce que** le médicament est un médicament pour le traitement de maladies obstructives artérielles ou coronariennes périphériques.

26. Utilisation selon la revendication 24, **caractérisée en ce que** le médicament sert de médicament pour le traitement de troubles démentiels d'origine vasculaire.

27. Utilisation selon l'une des revendications 23 à 26, **caractérisée en ce que** le médicament est une solution injectable.
